# FASCICULE DE BREVET EUROPEEN

(11) **EP 0 391 769 B1**
(45) Date de publication et mention de la délivrance du brevet: **30.03.1994**
(21) Numéro de dépôt: 90400824.0
(22) Date de dépôt: 27.03.1990
(51) Int. Cl.: A61K 31/695

(54) **Produit thérapeutique à base de composé organique du silicium et de polyamine polycarboxylée, particulièrement utile pour le traitement de l'athérome**
Therapeutisches Produkt auf der Basis von organischer Silicium-Verbindung und polycarboxyliertem Polyamin, nützlich insbesondere für die Behandlung des Atheroms
Therapeutical product based on an organic silicon compound and polycarboxylated poly-amine, particularly useful for treatment of atherome

(30) Priorité: 07.04.1989 FR 8904577
(43) Date de publication de la demande: 10.10.1990
(73) Titulaire: Gueyne, Jean, MC-98000 Monte-Carlo (MC); Seguin, Marie-Christine, MC-98000 Monte-Carlo (MC); Crussol, Gilbert H., F-75008 Paris (FR)
(72) Inventeur: Gueyne, Jean, MC-98000 Monte-Carlo (MC); Seguin, Marie-Christine, MC-98000 Monte-Carlo (MC); Crussol, Gilbert H., F-75008 Paris (FR)
(74) Mandataire: Bonneau, Gérard

(56) Documents cités:
- EP-A- 0 281 435
- FR-M- 6 871
- GB-A- 955 969
- La nomenclature en chimie organique, Noel Lozac'h, Masson et Cie Editeurs, 1967, Paris, FR, p. 70-71

## Description

La présente invention se rapporte à un produit thérapeutique formé par l'association d'un dérivé organique du silicium avec une polyamine porteuse de chaines hydrocarbonées comportant des groupes carboxyliques. Elle comprend notamment de telles associations, dans lesquelles la polyamine est un chélatant de différents métaux, en particulier du calcium. Le produit thérapeutique se trouve, plus spécialement, sous la forme d'une solution aqueuse, destinée à l'administration parentérale aux humains et aux animaux. Il est utile surtout au traitement de diverses affections vasculaires, particulièrement pour la réduction des plaques athéromateuses, inhibition de la peroxydation lipidique, stimulation de la régénération de fibres élastiques et la normalisation des métabolismes cellulaires.

De nombreuses substances, médicamenteuses, agissent sur le système vasculaire comme veinotoniques et vasculoprotecteurs en abaissant la perméabilité capillaire ; certaines exercent une action spasmolytique ou/et vasodilatatrice. Parmi les substances ainsi utilisées, on connaît, depuis longtemps, les flavonoïdes, saponosides, rutines et autres composés à activité vitaminique P, extraits de différentes plantes, les adénosine phosphate, naphtidrofuryl, buphénine, escine et autres. En général, elles améliorent la résistance des vaisseaux, mais ne font pas régénérer la paroi de ceux-ci. C'est seulement en 1974 qu'a paru sur le marché un médicament capable de stimuler la régénération des tissus conjonctifs ; ce progrès, particulièrement important pour le traitement des artérites, fut obtenu grâce à l'emploi de silanols injectables par voie I.V. ou I.M. Ainsi est-il devenu possible de régénérer les parois artérielles par environ 20 à 40 injections de 0,05 g de salicylate de méthyl-silane-triol, à raison de 2 injections par semaine, soit en 10 à 20 semaines, mais le plus souvent en environ 6 mois.

Or, l'expérience récente sur des applications thérapeutiques de dérivés organiques du silicium, a révélé ce fait complètement imprévu que la régénération de tissus conjonctifs peut être réalisée encore plus rapidement et de façon plus poussée, lorsque le silanol utilisé est combiné avec une polyamine aliphatique porteuse, dans ses chaînes hydrocarbonées, de plusieurs groupes carboxyliques, dont certains peuvent être neutralisés par un cation métallique.

On arrive ainsi au résultat remarquablement amélioré de rendre à la vie normale, par un traitement de 2 à 4 mois seulement, les malades dont le système vasculaire n'a pu être amélioré par aucun autre traitement. De plus, le produit thérapeutique suivant l'invention se prête particulièrement bien à l'administration par perfusion, ce qui se traduit par une efficacité accrue.

Le produit suivant l'invention, comprend un ou plusieurs silanols ou/et siloxanes, auxquels est associé un composé de formule 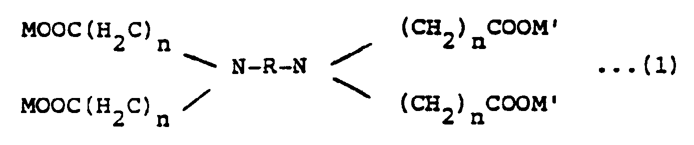
où n est 1 à 4, R est une chaîne (CH₂)ₘ, 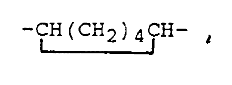
ou bien 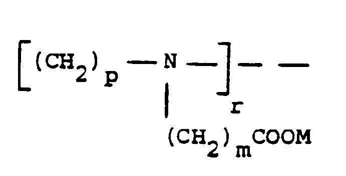
m étant 1 à 12, p 2 à 4 et r 1 à 3, tandis que M et M', semblables ou différents, représentent chacun une valence de H, de métal alcalin ou alcalino-terreux, ou de Zn, une partie des M ou/et M' pouvant être des H, caractérisé en ce que ce produit renferme au moins 0,2 atome de silicium par groupe carboxy -COO-.

De préférence, les proportions des réactifs sont telles qu'il y ait 0,2 à 2 atomes de Si par groupe carboxy-COO dans le produit, ou, mieux encore, 0,3 à 1 Si par -COO présent. La forme préférée de ce produit est une solution aqueuse.

Le plus souvent, le composé du silicium est introduit dans la solution suivant l'invention sous la forme d'un sel de métal alcalin ou alcalino-terreux, car c'est sous une telle forme qu'on est obligé de prendre un silanol ou un siloxane pour l'avoir à l'état non polymérisé. La proportion de cation alcalin ou alcalino-terreux est de 0,5 à 1,5 valences par atome de Si, et surtout 1 valence/1 Si.

Pour l'application thérapeutique, la solution aqueuse, suivant l'invention doit être faiblement acide ; il convient que son pH soit de 3,5 à 6,5 et - de préférence - de 5 à 5,5. Il s'en suit que, par rapport à la formule (1), donnée plus haut, le nombre de cations H, M et M' varie, dans la solution, selon la force de l'acide, afin que les limites de pH ci-dessus soient respectées.

Les polyamines polycarboxylées de formule (1) les plus courantes, utilisables suivant l'invention, citées ici à titre d'exemples sont :
E.D.T.A. -- acide éthylène diamine tétracétique
R étant -CH₂CH₂- n=1
C.D.T.A. -- acide trans-1,2 cyclohexane diamine tétracétique
R étant 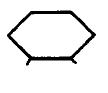
n=1
T.T.H.A. -- acide triéthylène tétramine hexaacétique
R étant 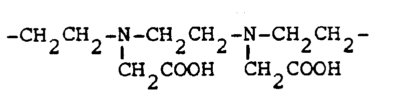
n=1
D.T.P.A. -- acide diéthylène triamine penta-acétique
R étant 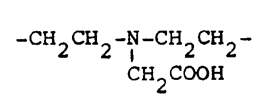
n=1

Le premier de ces composés, l'E.D.T.A., est le plus courant ; il est employé dans l'industrie, surtout comme chelatant du calcium ; son sel disodique est appliqué, par voie intraveineuse, au traitement de l'athérosclérose. Ce sel agit en arrêtant l'évolution et la formation de la plaque athéromateuse, mais il ne stimule nullement la régénération du tissu artériel ; il est donc surprenant que son association avec un silanol ou un siloxane se traduise par l'accentuation de l'action régénératrice de ces composés organiques du Si.

La solution thérapeutique suivant l'invention renferme, en tant que composé silicié, un silanol du type R'ₙSi(OH)₍₄₋ₙ₎ où n est 1 à 3 et R' un groupe hydrocarboné de préférence un alkyle en C₁ à C₁₈ et surtout en C₁ à C₆ ; d'autre part, le dérivé silicié peut être un siloxane, tel que par exemple 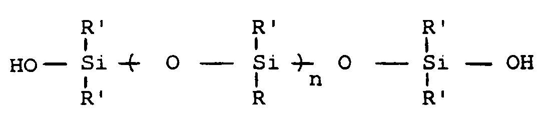
où R' est un groupe hydrocarboné, de préférence alkyle en C₁ à C₁₈ et n un nombre de 0 à 20 et de préférence 0 à 4.

Bien que la concentration de la solution aqueuse en polyamine polycarboxylée et en composé du silicium puisse varier entre de larges limites, elle est en général de 0,5 à 30 mmoles de polyamine polycarboxylée avec 2 à 60 mmoles de dérivé silicié, par litre, le rapport molaire entre ces deux sortes de composés associés étant conforme à ce qui est indiqué plus haut. Dans le cas particulier du complexe, ainsi formé entre l'E.D.T.A. et le méthyl silane triol, les concentrations pondérales en ce complexe varient par exemple entre 1 et 20g par litre, et de préférence entre 3 et 5 g/litre.

La solution thérapeutique suivant l'invention, en particulier le liquide pour perfusions, peut être préparée par mélange de deux portions aqueuses, dans lesquelles on a délayé ou dissous au préalable, respectivement, la polyamine polycarboxylée et le composé silicié. Le pH de la solution est ensuite amené à la marge requise, à la manière connue en soi, notamment par addition de solution de soude, sous agitation. Afin d'éviter des changements de pH trop grands, il est recommandable de régler d'avance le pH de la solution de polyamine polycarboxylée à une valeur au moins voisine de celle que l'on doit avoir à la fin ; cela peut revenir à utiliser un sel alcalin, alcalino-terreux ou zincique, dans lequel les groupes carboxyliques sont en partie neutralisés par le cation correspondant.

Une condition importante, pour les applications thérapeutiques des solutions suivant l'invention, est l'isotonie de ces solutions. C'est pourquoi la concentration molaire en le complexe silicié, et en sels éventuellement ajoutés, est réglée de façon à être égale à celle qui existe dans le plasma sanguin de l'homme ou de l'animal devant recevoir la solution thérapeutique.

Bien que l'association des deux composés, suivant l'invention, seule, procure l'effet de régénération des tissus artériel et veineux, par cytostimulation des fibroblastes qui produisent des fibres élastiques et collagéniques de bonne qualité, il est recommandable - pour des raisons pharmacologiques - d'inclure dans la nouvelle solution thérapeutique certains adjuvants. Ainsi, est-il bon d'y ajouter de l'héparine, un anesthésique local, comme lidocaïne ou procaïne, des vitamines, en particulier l'acide ascorbique et celles du groupe B, des oligo-éléments et - comme mentionné plus haut - un électrolyte pour assurer l'isotonie, si la concentration en molécules de complexe polyamine polycarboxylée-composé de Si ne produit pas la pression osmotique suffisante.

Suivant l'état des malades à traiter, il peut être utile ou nécessaire d'adjoindre à la solution d'autres éléments thérapeutiques, compatibles avec les complexes suivant l'invention.

De toute manière, même en l'absence d'autres adjuvants la solution suivant l'invention exerce sur les vaisseaux une action lipolytique, permettant de réduire les plaques athéromateuses ; elle a un effet d'inhibition de la peroxydation lipidique, ce qui procure une protection contre la cytotoxicité de radicaux libres ; comme déjà exposé plus haut, un avantage important de cette solution résiste dans l'intensification cytostimulante du composé silicié ; en outre, on constate la normalisation des métabolismes cellulaires.

A titre d'exemple voici une formule pour 1000 ml de solution suivant l'invention, convenant bien aux perfusions :
3 à 5 g de complexe polyamine polycarboxylée/silanol
0,2 g MgCl₂ ou MgSO₄
0,2 g lidocaïne ou procaïne
1000 à 5000 unités d'héparine
4 à 20g d'acide ascorbique

Dans ce qui précède, on a décrit la forme du produit convenant le mieux à l'administration parentérale, notamment par perfusion. Ce produit peut cependant se présenter sous d'autres formes : liquides, solides pâteuses ou en émulsion. La caractéristique commune de toutes ces formes est l'association d'une polyamine de formule (1) avec un silanol ou/et siloxane.

Ainsi peut-on conserver et transporter le produit à l'état d'une solution aqueuse de complexe suivant l'invention, tenant en suspension fine la polyamine carboxylée de formule (1) non dissoute et du silanol ou/et siloxane. Cette suspension aqueuse peut contenir, par exemple, 5 à 20 fois plus de produit actif que la solution définitive, utilisée pour l'injection au malade ; elle est diluée avec de l'eau distillée, au moment de l'emploi, de façon à présenter le titre compris entre les limites indiquées plus haut.

Une autre forme de présentation du produit suivant l'invention est un mélange pulvérulent des deux composés, enfermé dans un contenant étanche. Ainsi peut-on avoir des ampoules ou flacons, renfermant, chacun, une dose injectable ; celle-ci est dissoute dans une dose d'eau distillée, accompagnant l'ampoule ou le flacon, au moment de l'administration du médicament.

L'invention est illustrée, par les exemples qui suivent.

### EXEMPLE 1

### Préparation d'une solution thérapeutique à base de l'acide éthylène diamine tétracétique (EDTA) et de monométhyl silane triol (MST).

Dans 500 ml d'eau distillée, on délaye 6,4 g, soit 21,9 mmoles, d'EDTA en poudre fine. Sous vive agitation, on ajoute lentement 400 ml de solution aqueuse de 3,5 g, c'est-à-dire 37,2 mmoles de CH₃Si(OH)₃ renfermant 37,2 mmoles de NaOH. Le pH du mélange est amené à la valeur de 5 par addition d'une solution aqueuse de NaOH. Le volume final est de 2000 ml et il contient 6,4 + 3,5 = 9,9 g de complexe EDTA-silanol. Le rapport molaire Si/EDTA est de 37,2/21,9=1,7 ce qui correspond à 1,7:4=0,424 atomes Si par groupe COOH de l'EDTA utilisé.

### EXEMPLE 2

Les opérations sont similaires à celle de l'exemple 1, mais l'EDTA est pris sous la forme de son sel disodique à raison de 7,36 g (21,9 mmoles), tandis qu'à la place du MST on a utilisé 5,06 g de C₄H₉Si(OH)₃ soit 37,2 mmoles. Le pH est ajusté à 5,08 et le volume du mélange est complété à 2000 ml qui contiennent alors 7,36 + 5,08 = 12,44 g de complexe au silicium, les rapports molaires étant les mêmes que dans l'exemple 1.

### EXEMPLE 3

On répète l'exemple 1, sauf que la solution de méthyl silane triol (MST) contient 46,5 mmoles de NaOH (1,86 g) ce qui fait qu'à la fin il faut beaucoup moins de soude pour arriver au pH5.

### EXEMPLE 4

La préparation est effectuée comme dans l'exemple 3, le MST étant remplacé par la même quantité de diméthyl silane diol (CH₃)₂Si(OH)₂.

### EXEMPLE 5

### Solution thérapeutique à base de l'acide 1,2-cyclohexane diamine tétracétique, C.D.T.A. et de monométhyl silane triol.

Dans 500 ml d'eau distillée, on disperse 7,6 g d'acide C.D.T.A., soit 21,9 mmoles ; sous bonne agitation, on ajoute 6,9 g de CH₃Si(OH)₃ (soit 74 mmoles) en solution dans 400 ml d'eau renfermant 3g de NaOH (75 mmoles). On complète le volume total à 2000 ml en ajustant son pH à 4,9. On a ainsi un complexe comprenant 3,38 moles de silanol par mole de CDTA, soit 0,844 atomes Si par groupe COOH de cette diamine tétracarboxylée.

### EXEMPLE 6

La solution est préparée de la même façon qu'à l'exemple 5, sauf que le silanol utilisé est le diméthyl silane diol.

### EXEMPLE 7

On procède comme dans l'exemple 5, mais les réactifs employés sont :
acide triéthylène tétramine hexacétique T.T.H.A. - 10,8 g soit 21,9 mmoles, et diméthyl silane diol (CH₃)₂Si(OH)₂ 6,8g =74mmoles.
Volume total 2000 ml. pH final 5,1
3,38 moles de silanol par mole de TTHA, soit 0,56 atome de Si par groupe -COOH de celui-ci.

### EXEMPLE 8

Dans la formule de l'exemple 7, le silanol utilisé est le monoéthylsilane triol C₂H₅Si(OH)₃ à raison de 15,6 g soit 144,5 mmoles, ce qui représente 6,6 atomes Si par mole de TTHA, ou 1,1 Si par -COOH.

### EXEMPLE 9

La solution est préparée comme dans l'exemple 1, mais avec 21,9 mmoles d'acide diéthylène triamine pentacétique, D.T.P.A., soit 8,6 g, au lieu de 6,4 g d'E.D.T.A. Il y a alors 0,34 atome Si par groupe -COOH.

### EXEMPLE 10

La composition de la solution est, par litre, de 16 mmoles de D.T.P.A., soit 6,3 g, avec 64 mmoles de diméthyl silane diol, (CH₃)₂Si(OH)₂ ou 5,9 g. Le rapport molaire silanol/D.T.P.A. est de 4, soit 0,8 atome Si par groupe -COOH.

### APPLICATIONS CLINIQUES

Selon le degré et la nature de l'affection, le mode de traitement, notamment la concentration en complexe polyamine polycarboxylée/silanol ou siloxane, et le mode d'administration, en particulier injection IM, IV ou perfusion, peuvent ou doivent varier. Dans les traitements dont les résultats furent très favorables, on opérait par perfusions lentes, d'environ 2 à 4 heures, à raison de 2 ou 3 par semaine, le volume liquide utilisé étant chaque fois de 500 à 1000 ml. Celui-ci renfermait en solution environ 3 à 5g de complexe actif, accompagné des adjuvants indiqués plus haut, avant les exemples.

Dans les traitements dont les rapports suivent, chaque perfusion était faite avec 5g de complexe dans le volume précisé dans le rapport, renfermant également 0,2 g de MgCl₂ 0,2 g de lidocaïne, 10 g d'acide ascorbique et 3000 unités d'héparine.

En tête de chaque rapport figure son numéro d'ordre en chiffres romains, les initiales du malade, son âge et son sexe.

### I - P.M. 61 F

Malade atteinte d'une angine de poitrine. Traitée avec le complexe E.D.T.A. - monométhylsilane triol de l'exemple 1. 1° 2° 3° perfusion, 500 ml, durée 4 heures, 4° 5° perfusion 600 ml, durée 3,5 heures, 6° et 7° perfusion, 600 ml, durée 2,5 heures, ensuite treize perfusions de 400 ml d'une durée de 2 heures. Amélioration progressive avec disparition des douleurs d'angine de poitrine après 8ème perfusion.
Au total 20 perfusions en deux mois.
Amélioration de l'ouïe, ainsi que l'arrêt de l'écoulement mucoïde nasal qui durait depuis 30 ans à la suite d'un coup sur la tête.
L'examen Doppler des artères carotides confirme l'amélioration constatée par la patiente ; elle ne prend plus aucun médicament.

### II - D.S. 64 F

Artérite des membres inférieurs. La patiente se présente à la clinique marchant très difficilement, même avec une canne, malgré 7 mois de traitement classique au naftidrofuryl (Praxilène).
La chirurgie vasculaire vient de lui être refusée à cause du mauvais état de ses artères. La pression artérielle a toujours été voisine de 18/13.
La malade parait 15 ans plus jeune après quatre mois de traitement avec le complexe T.T.H.A. - diméthylsilane diol de l'exemple 7, à raison de 500 ml par perfusion d'une durée de trois heures, une fois par semaine. Elle peut maintenant faire, seule et sans canne, plus d'un kilomètre.
Sa pression artérielle est stabilisée à 14/10.

### III - A.R. 53 M

Claudication intermittente avec blocage total à la partie supérieure de la cuisse (artériographie), en dépit de 36 injections I.M. de Conjonctyl en 18 semaines.
Circulation collatérale relative.
On pratique 20 perfusions du complexe C.D.T.A. - monométhyl-silanetriol selon l'exemple 5. Les 5 premières sont faites tous les quatre jours à raison de 250 ml pendant 4 heures, les 5 suivantes de 300 ml tous les 3 jours, pendant 3 heures ; les 10 suivantes : tous les 3 jours à raison de 500 ml pendant 2,5 heures.
Après 10 semaines de ce traitement, une reprise normale de la mobilité est notée ; la malade peut monter les escaliers sans difficulté.

### IV - P.G. 58 M

Infarctus et pontage cardiaque. Hypertension suivie d'une hémorragie cérébrale. Doppler pessimiste. Etat grave malgré 1 an de soins classiques.
30 perfusions sont pratiquées à raison de deux par semaine de complexe E.D.T.A. - diméthylsilanediol selon l'exemple 4. Les médicaments sont réduits graduellement après la sixième perfusion, tandis que l'hypertension diminue.
Après 4 mois, on constate une amélioration sensible du Doppler au niveau des carotides, tendant vers la normalité ; une rechute est devenue improbable.

### V. B.K. 57 F

Une hémorragie cérébrale 9ans auparavant a laissé persister une paralysie considérable du côté droit. Le Doppler révèle un ralentissement du flux sanguin au niveau carotides.
Une série de 25 perfusions du complexe T.T.H.A. - monométhyl-silanetriol selon l'exemple 8 sont prescrites à raison de 500 ml pendant 4 heures tous les jours.
On note une nette amélioration à la suite de la 6 ème perfusion, trois mois de traitement.
On recommande au patient de débuter la physiotérapie, car la régénération nerveuse parait possible à la suite de l'amélioration de la vascularisation.

### VI - S.T. 82 F

Malgré 1 an de médication classique, suivie de 32 I.V. de Conjonctyl, en 4 mois, une claudication intermittente ne permet à ce malade que quelques 20 mètres d'autonomie.
Affection cardiaque et perte importante de potassium.
La posologie de l'exemple précédent est instaurée avec le complex- D.T.P.A. - diméthylsilanediol selon l'exemple 10. Une série de 25 perfusions sont effectuées à raison de 500 ml pendant 4 heures tous les 4 jours. Trois mois de traitement.
La fonction rénale insuffisante ne permet qu'un demi-dosage du potassium durant les cinq premières perfusions.
La fonction rénale s'est par la suite améliorée. Le malade a doublé son périmètre de marche.

### VII - P.B. 55 F

Les chevilles sont très enflées à la suite de la moindre marche. Obèse, bien que mangeant très peu. A constamment froid et manque d'énergie.
Le Doppler ne révèle que des anomalies infimes dans les carotides. Aucun examen particulier des valves veineuses.
Après 24 perfusions du complexe C.D.T.A. - diméthyl silane-diol selon l'exemple 6, tous les 5 jours de 500 ml pendant 3 heures, éliminent la rétention hydrique des chevilles.
Après quatre mois de traitement, la malade a perdu du poids et se sent bien.

### VIII - D.J. 63 M

Après 16 mois de traitement à la Fonzylane, le malade ne pouvait faire qu'une vingtaine de pas, sans qu'une violente douleur l'oblige à s'arrêter, les deux jambes étant également affectées.
24 perfusions du complexe E.D.T.A. - diméthylsilanediol selon l'exemple 4, sont faites de 400 ml tous les 3 jours durant 3 heures.
Le malade peut effectuer 2 km deux mois après la fin de ce traitement.

### IX - B.A. 58 M

Circulation artérielle des jambes obtenue seulement avec le concours de l'action cardiaque, bien que le Doppler semble révéler un système artériel principal intact.
14 mois de traitement aux rutosides, et 2 mois au salicylate de méthyl silane triol, n'ont pas changé la situation.
On applique alors 400 ml du complexe E.D.T.A. - monométhylsilanethiol selon l'exemple 2, tous les deux jours pendant 4 heures avec un total de 20 perfusions.
Durée du traitement deux mois et demi.
Après six perfusions, le malade peut enfin dormir, pour la première fois après plusieurs semaines d'insomnie. Le Doppler montre une diminution de l'obstruction aortoiliaque. En moins de deux mois de traitement, on constate une amélioration considérable.

### X - B.R. 40 M

Proposition de chirurgie cardiaque pour soulager le malade d'une douleur d'angine de poitrine occasionnelle, mais extrêmement violente.
Prescription de 25 perfusions du complexe D.T.P.A. - monométhylsilane triol selon l'exemple 10, de 300 ml pendant 4 heures, tous les 4 jours. Trois mois et demi de traitement, mais les douleurs résiduelles n'ont pas réapparu après la 14ème perfusion (2 mois).

### XI - M.G. 79 F

Troubles oculaires, perte d'équilibre, perte presque totale de la vue, pertes de connaissance, malaises généraux et constants rendent la vie de cette patiente insupportable.
Les traitements classiques n'apportent que de légères améliorations momentanées.
Des perfusions sont commencées de toute urgence à raison de 500 ml du complexe T.T.H.A. - monométhylsilanetriol selon l'exemple 8, pendant 4 heures. Le premier jour, la malade étant fatiguée, les perfusions suivantes furent de 250 ml, une fois par semaine, pendant 3 mois. Au bout de ce temps, on observe la disparition de tous les symptomes.

### XII - H.A. 62 M

Ce malade est réveillé trois fois par nuit par des douleurs d'angine de poitrine. Les antiangoreux habituels, pendant 6 mois, ne donnent que des résultats passagers.
Le Doppler bidirectionnel montre des refoulements qui font penser à la valve aortique.
On administre alors 500 ml de complexe T.T.H.A. - diméthylsilanediol selon l'exemple 7, durant 5 heures et pendant 4 mois à raison de deux perfusions par semaine.
Après la seconde perfusion, les douleurs nocturnes disparaissent et l'amélioration des symptômes diurnes débute après la 4ème perfusion.
L'examen Doppler à la fin du traitement révèle une normalisation presque complète.

### Expérimentation animale

### XIII

Un hongre de 16 ans, qui vient de mordre cruellement une éventuelle acheteuse, est sauvé de la boucherie par son dernier acheteur après 7 années de service et de sévices dans un cercle hippique de la région parisienne ; cheval fatigué, boîteux, hargneux, dangereux, inutilisable et triste.
On lui applique une perfusion de 4 heures avec 1,5 litres de soluté, deux fois par semaine, pendant plus de trois mois, assortie d'une présence humaine et surveillance pendant toute la durée des perfusions.
Cesse presque de boîter, après la troisième perfusion ; après la septième ne boîte plus même lorsqu'on le fait tourner à la longue à main gauche sur un cercle court, (les pires conditions pour son ancienne boiterie).
Le traitement est poursuivi pour un total de 28 perfusions en trois mois avec le soluté d'E.D.T.A./méthylsilane triol selon l'exemple 3.
Le cheval est alors en pleine forme et son entraînement a pu reprendre sur un rythme normal.

### XIV

Hongre de 8 ans, arrêté de concours hippiques depuis un an, boîterie rebelle à tous traitements. Il cesse de boîter même sur cercle court à main droite, grâce au traitement avec le soluté de l'exemple 5, qui comportera 24 perfusions, en trois mois. Ce cheval, qui montrait une grande paresse en plus de sa boîterie, fait à présent quatre fois le tour du paddock au grand galop, lorsqu il est mis en liberté ; touchard, il fait maintenant des parcours sans faute, avec courage. Un jeune entier de deux ans avec lequel il paturait sans incidents, lorsqu'il était malade, l'attaque et le mord maintenant. Il doit maintenant pâturer seul.

### XV

Entier de 7 ans, acheté trop cher récemment, après une visite vétérinaire favorable, se met à boîter après le premier concours hippique ; les nouveaux propriétaires, novices dans le monde du cheval, apprennent alors que le cheval était en permanence dopé aux antiinflammatoires par son précédent propriétaire, pour pouvoir concourir.
La douleur se situe sur la partie externe d'un sabot antérieur, comme prouvé par anesthésie locale. Dés la première séance de perfusion, on remarque que le sabot gauche, sensible, était froid, comparé au sabot homologue, et le restait encore durant les deux premières heures de perfusion ; il devenait ensuite très chaud et le restait jusqu'à la fin.
Durant les séances suivantes, la différence de température s'est progressivement atténuée et égalisée, la température des sabots est devenue constante.
Ce traitement, avec le soluté de l'exemple 8, comporta 26 cessions, en trois mois, très difficile avec un entier, a conduit au résultat remarquable que le cheval ne boîte plus deux mois après la fin du traitement et a repris un travail normal.

### XVI - L.A. 44 M

Hospitalisé pour artérite du membre inférieur gauche au stade II. Périmètre de marche à 150 mètres. Ces troubles ont débuté brutalement par une claudication intermitente du mollet gauche.
L'artériographie montre ; à droite, d'importantes surcharges de l'iliaque primitive et une thrombose de l'iliaque interne ; à gauche, obstruction quasi totale de l'iliaque primitive, thrombose de l'iliaque interne et sténose de la fémorale commune. On pratique, pendant 2 mois, à raison d'une fois par semaine, des perfusions de 3 heures chacune, avec 500 ml d'une solution aqueuse à 3,2 g d'E.D.T.A. et 0,36 g de CH₃Si(OH)₃ par litre, amenée à pH 5.
C'est une solution analogue à celle de l'exemple 1 mais renfermant seulement 0,088 atomes Si par groupe -COOH de l'EDTA utilisé.
Après ce traitement de 2 mois, le périmètre de marche du malade est passé à 400-500 mètres.
Un mois de plus de traitement n'apporte pas d'amélioration de son périmètre de marche.
Un traitement de deux mois supplémentaires est décidé, avec la solution de l'exemple 1, c'est-à-dire à 0,424 atome Si par groupe -COOH dans les mêmes conditions que plus haut.
Le périmètre de marche devient alors illimité, même en cas d'efforts violents et tous les pouls sont perçus.

### XVII - B.A. 72 M

Artérite des deux membres inférieurs au stade III. Le périmètre de marche est de 150 mètres, sans douleurs de décubitus, mais avec de petites plaques de nécrose au niveau du pied gauche.
L'artériographie montre une surcharge diffuse aorto-iliofémorale avec thrombose des iliaques internes et thrombose fémoro-poplitée bilatérale.
Un traitement par perfusions de 500 ml de solution de T.T.H.A. similaire à celle de l'exemple 7, mais avec seulement 0,05 atomes Si par groupe -COOH, est institué, à raison de 3 heures une fois par semaine.
Le périmètre de marche passe de 80 mètres à 300 mètres.
Le traitement est poursuivi pendant un mois ; le périmètre de marche passe à 350 mètres.
Il est alors décidé de traiter ce malade pendant un mois, au même rythme que précédemment avec la solution de l'exemple 7, contenant 3,38 moles de (CH₃)₂Si(OH)₂ par mole de T.T.H.A., soit 0,563 atomes Si par groupe -COOH. Le périmètre de marche passe alors à 600 mètres et, après un mois de plus de traitement, à 850 mètres. On observe de plus la cicatrisation des plaques de nécrose.

Les rapports cliniques XVI et XVII prouvent que des résultats vraiment bons s'obtiennent seulement lorsque la solution employée contient au moins plusieurs dixièmes d'atome de Si par groupe -COO de la polyamine polycarboxylée utilisée.

### CARACTERISATION DES COMPLEXES SUIVANT L'INVENTION.

Les solutions, préparées dans les exemples 1 à 10, étaient soumises à des mesures de l'absorption de la lumière pour différentes longueurs d'onde de celle-ci. Ces mesures étaient effectuées comparativement avec celles de solutions renfermant seulement de la polyamine polycarboxylée correspondante ou du silanol correspondant seul.
Les mesures avaient lieu à la température ambiante. On se servait de l'appareil connu sous la dénomination commerciale "KONTRON INSTRUMENTS" ("UVIKON 930"). Le graphique annexé illustre les variations relatives de l'absorption de la lumière (ordonnées) en fonction des longueurs d'onde de la lumière en nm (abscisses).
Courbe 1 correspond à la solution finale de l'exemple 1 c'est-à-dire complexe EDTA-silanol.
Courbe 2 est celle d'une solution aqueuse contenant seulement de l'EDTA à la même concentration que la solution de la courbe 1, soit 3,2 g/litre.
Courbe 3 est obtenue avec une solution aqueuse de silanol CH₃Si(OH)₃ seul, à la même concentration que la solution 1, c'est-à-dire 1,75 g/l.
On constate des différences nettes d'absorption qui indiquent pour le complexe formé, d'après la courbe 1, des valeurs supérieures à celles des courbes 2 et 3. Ainsi pour le complexe EDTA-silanol (courbe 1) trouve-t-on un maximum au voisinage de la longueur d'onde, de 220 nm ; pour l'EDTA seul (courbe 2) le maximum est situé vers 210 nm, et pour le silanol seul (courbe 3) vers 200 nm.

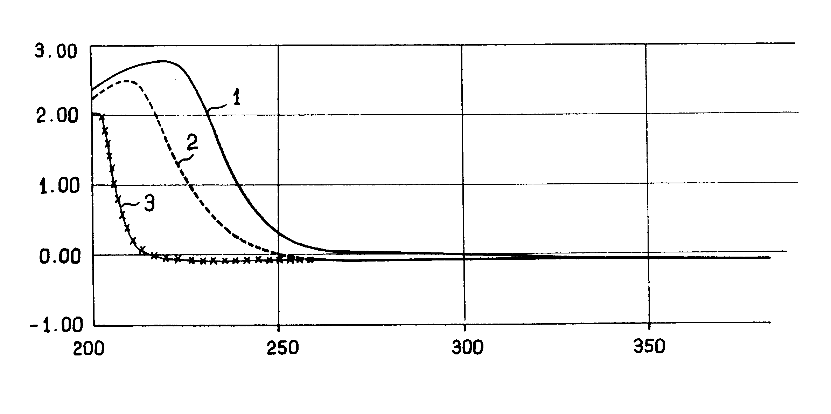

## Revendications

1. Produit thérapeutique contre affections vasculaires, comprenant un silanol ou un siloxane et une amine du type
[MOOC(CH₂)ₙ]₂N-R-N[(CH₂)ₙCOOM']₂
où n est 1 à 4, R est une chaîne (CH₂)ₘ, 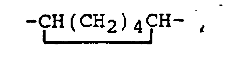 ou bien 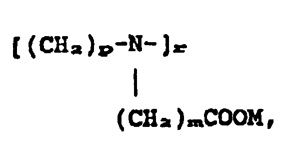 m étant 1 à 12, p 2 à 4 et r 1 à 3, tandis que M et M' semblables ou différents, représentent chacun une valence de H, de métal alcalin ou alcalino-terreux, ou de Zn, une partie des M ou/et de M' pouvant être des H, caractérisé en ce que ce produit renferme au moins 0,2 atome de silicium par groupe carboxy -COO-.

2. Produit suivant la revendication 1, caractérisé en ce qu'il renferme 0,2 à 2 atomes Si par groupe -COO-.

3. Produit suivant la revendication 1 ou 2, caractérisé en ce qu'il renferme 0,3 à 1 atome Si par groupe -COO-.

4. Produit suivant une des revendications 1 à 3, caractérisé en ce que le silanol est du type Rₙ'-Si(OH)₍₄₋ₙ₎ où n est 1 à 3, et R' un groupe hydrocarboné, de préférence un alkyle en C₁ à C₁₈.

5. Produit suivant la revendication 4, caractérisé en ce que R' est un alkyle en C₁ à C₆.

6. Produit suivant une des revendications 1 à 3, caractérisé en ce que le siloxane est du type 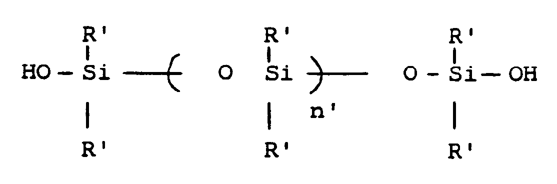 où R' est un groupe hydrocarboné, de préférence alkyle en C₁ à C₁₈ et n' un nombre de 0 à 20 et de préférence de 0 à 4.

7. Produit suivant une des revendications précédentes, caractérisé en ce qu'il se présente sous la forme d'une solution aqueuse de pH 3,5 à 6,5 , et de préférence de 4 à 5,5.

8. Produit suivant la revendication 7, caractérisé en ce que la solution aqueuse renferme 1 à 20g de l'association polyamine-polycarboxylée + silanol ou siloxane par litre, et - plus particulièrement - 3 à 5g/litre.

9. Produit suivant la revendication 7 ou 8, caractérisé en ce que la solution aqueuse est isotonique avec le plasma sanguin, humain ou animal.

10. Application du produit suivant la revendication 9 à la préparation d'un liquide pour injection parentérale, notamment perfusion, caractérisé en ce qu'il contient un sel minéral, un anesthésique local, une ou plusieures vitamines et de l'héparine.

## Claims

1. Therapeutic product for against vascular diseases, comprising a silanol or a siloxane and an amine of the kind
[MOOC(CH₂)ₙ]₂N-R-N[(CH₂)ₙCOOM']₂
where n is 1 to 4, R is a chain (CH2)m, 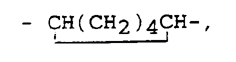 or 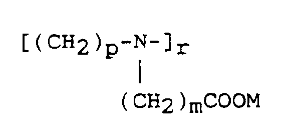 m being 1 to 12, p 2 to 4 and r 1 to 3, whereas M and M', similar or different, each represent a valency H of an alkaline or alkaline-earth metal or of Zn, it being understood that a part of M or/and M' can be H; characterized in that this product contains at least 0,2 atom of silicium per carboxy group -COO-.

2. Product according to claim 1, characterized in that it contains 0,2 to 2 atoms of Si per carboxy group -COO-.

3. Product according to claim 1 or 2, characterized in that it contains 0,3 to 1 atom of Si per carboxy group -COO-.

4. Product according to claim 1 to 3, characterized in that the silanol is of the kind R'ₙ-Si(OH)₍₄₋ₙ₎ wherein n is 1 to 3, and R' is a hydrocarbon group, preferably a C₁ to C₁₈ alkyl.

5. Product according to claim 4, characterized in that R' is a C₁ to C₆ alkyl.

6. Product according to claim 1 to 3, characterized in that the siloxane is of the kind 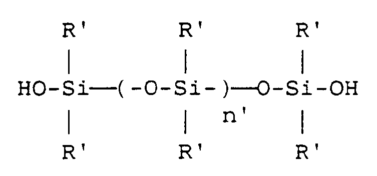 ou R' is a hydrocarbon group, preferably a C₁ to C₁₈ alkyl, and n' a number from 0 to 20 and preferably from 0 to 4.

7. Product according to one of the previous claims, characterized in that it is in the form of an aqueous solution having a pH ranging from 3,5 to 6,5, and preferably from 4 to 5,5.

8. Product according to claim 7, characterized in that the aqueous solution contains 1 to 20g of the association polycarboxylated-polyamine + silanol or siloxane per liter, and, more particularly, 3 to 5g/liter

9. Product according to claim 7 or 8, characterized in that the aqueous solution is isotonic with blood plasma, human or animal.

10. Application of the product according to claim 9 to the preparation of a liquid for parental injection, infusion in particular, characterized in that said liquid contains a mineral salt, a local anaesthetic, one or more vitamins and heparin.

## Patentansprüche

1. Therapeutisches Produkt gegen Venenbeschwerden, umfassend ein Silanol oder ein Siloxan und ein Amin vom Typ
[MOOC(CH₂)ₙ]₂N-R-N[(CH₂)ₙCOOM']₂
worin n eine Zahl von 1 bis 4 bedeutet, R eine Kette (CH₂)ₘ, 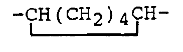 oder 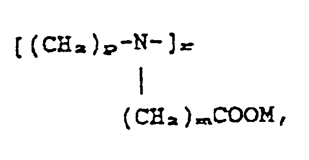 bedeutet, m eine Zahl von 1 bis 12 darstellt, p eine Zahl von 2 bis 4 darstellt und r eine Zahl von 1 bis 3 darstellt, während M und M', die gleich oder verscheiden sein können, jeweils eine Wertigkeit von H, einem Alkali- oder Erdalkalimetall oder von Zn darstellen, wobei ein Teil der Gruppen M oder/und M' H sein können, dadurch gekennzeichnet, daß dieses Produkt mindestens 0,2 Siliciumatome pro Carboxygruppe -COO- umfaßt.

2. Produkt nach Anspruch 1, dadurch gekennzeichnet, daß es 0,2 bis 2 Si-Atome pro -COO-Gruppe umfaßt.

3. Produkt nach Anspruch 1 oder 2, dadurch gekennzeichnet, daß es 0,3 bis 1 Si-Atom pro -COO-Gruppe umfaßt.

4. Produkt nach einem der Ansprüche 1 bis 3, dadurch gekennzeichnet, daß das Silanol vom Typ R'ₙ-Si(OH)₍₄₋ₙ₎ ist, worin n eine Zahl von 1 bis 3 bedeutet und R' eine Kohlenwasserstoffgruppe, vorzugsweise C₁- bis C₁₈-Alkyl, bedeutet.

5. Produkt nach Anspruch 4, dadurch gekennzeichnet, daß R' ein C₁- bis C₆-Alkyl bedeutet.

6. Produkt nach einem der Ansprüche 1 bis 3, dadurch gekennzeichnet, daß das Siloxan vom Typ 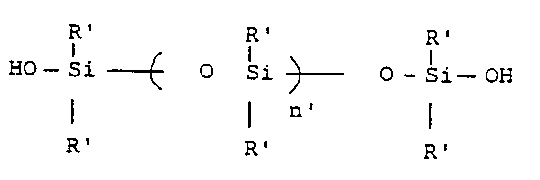 ist, worin R' für eine Kohlenwasserstoffgruppe, vorzugsweise für C₁- bis C₁₈-Alkyl, steht und n' eine Zahl von 0 bis 20, vorzugsweise von 0 bis 4, bedeutet.

7. Produkt nach einem der vorgenannten Ansprüche, dadurch gekennzeichnet, daß es in Form einer wäßrigen Lösung mit einem pH-Wert von 3,5 bis 6,5 und vorzugsweise von 4 bis 5,5 dargereicht wird.

8. Produkt nach Anspruch 7, dadurch gekennzeichnet, daß die wäßrige Lösung 1 bis 20 g eines Gemisches aus polycarboxyliertem Polyamin + Silanol oder Siloxan pro Liter, insbesondere 3 bis 5 g pro Liter, umfaßt.

9. Produkt nach Anspruch 7 oder 8, dadurch gekennzeichnet, daß die wäßrige Lösung mit menschlichem oder tierischem Blutplasma isoton ist.

10. Anwendung des Produkts nach Anspruch 9 zur Herstellung einer Flüssigkeit zur parenteralen Injektion, insbesondere einer Perfusion, dadurch gekennzeichnet, daß es ein Mineralsalz, ein Lokalanästhetikum, ein oder mehrere Vitamine und Heparin enthält.
